# EUROPEAN PATENT SPECIFICATION

(11) **EP 1 317 255 B1**
(45) Date of publication and mention of the grant of the patent: **06.10.2004**
(21) Application number: 01940817.8
(22) Date of filing: 21.06.2001
(51) Int. Cl.: A61K 9/48

(54) **DELIVERY CAPSULES**
VERABREICHUNGSKAPSELN
CAPSULES RENFERMANT DES SUBSTANCES MEDICAMENTEUSES

(30) Priority: 07.07.2000 WO PCT/GB00/02616; 11.04.2001 GB 0109089
(43) Date of publication of application: 11.06.2003
(73) Proprietor: Bioprogress Technology International, Inc., March, Cambridgeshire PE15 8QD (GB)
(72) Inventor: NOWAK, Edward, Zbygniew, Impington, Cambridge CB4 9YJ (GB)
(74) Representative: Hill, Justin John
(86) International application number: PCT/GB2001/002766
(87) International publication number: WO 2002/003968

(56) References cited:
- EP-A- 0 110 502
- US-A- 2 810 659

## Description

### Field of the Invention

This invention relates to a delivery capsule, that is, a capsule designed to retain and protect its contents until an intended site of delivery or conditions of delivery are encountered, at which point the capsule contents are released.

### Background to the Invention

Delivery capsules are well known and find particular application in the form of ingestible gelatin capsules for the delivery of accurately metered doses of pharmaceutical preparations and dietary supplements. Liquid preparations are typically encapsulated in soft gelatin capsules and particulate or powdered preparations are typically encapsulated in two part hard gelatin capsules. The capsules are designed to release their contents after ingestion, typically by solution of the capsule wall at a location in the digestive system of a consumer. By use of suitable capsule material such capsules can thus provide a means of administering a dose of a preparation at a desired appropriate site in the body. The finished capsules offer protection to the contents yet solubility within the body.

Document US-A-2 810 659 discloses a capsule shell made of plasticized hydroxypropyl methylcellulose.

### Summary of the Invention

In one aspect the invention provides a delivery capsule having an enclosing wall comprising a thermoplastic film of foamed modified cellulose material.

The modified cellulose material is preferably hydroxypropyl methyl cellulose (HPMC), which is a synthetic thermoplastic material that is a modified form of the naturally occurring polymer cellulose. Other modified cellulose materials include hydroxypropyl cellulose (HPC).

In a preferred aspect the invention thus provides a delivery capsule having an enclosing wall comprising a thermoplastic film of foamed hydroxypropyl methyl cellulose.

Modified cellulose materials are suitable for ingestion by humans, so the delivery capsules of the invention are suitable for human consumption.

The material is foamed, that is, the material includes a plurality of small openings, pockets or voids within the body of the material. The voids are typically filled with gas, commonly air, oxygen or carbon dioxide, but preferably nitrogen, although the voids may be empty. The voids should be small (typically having a maximum dimension in the range 1 to 100 microns) and are ideally substantially uniform in the size and are preferably reasonably uniformly dispersed through the material, for uniformity of properties. Foamed material may also be referred to as expanded material, gasified material or aerated material.

The void volume of the foamed material should be selected to give desired properties. Generally the void volume is in the range 20 to 60% by volume, typically being about 50% by volume.

Foamed modified cellulose materials, with a controlled desired void level, can be readily made, e.g. by mechanical entrainment of gas, such as nitrogen, in appropriate quantity in a liquid to be used for production of a cast film.

The film material typically includes a plasticiser to give desired properties of flexibility to the film, in known manner. Materials used as plasticisers include polyethylene glycol (PEG), monopropylene glycol, glycerol and also acetins, which are acetates of glycerol.

The film typically has a thickness in the range 50 to 200 microns, e.g. in the range 140 to 150 microns, with film thickness being controllable in known manner. Films of different thickness may be suited to different uses.

It is surprisingly found that the presence of voids in the foamed film results in the film rapidly starting to dissolve in the mouth of a consumer, possibly after only a few seconds of chewing or sucking, resulting into release into the mouth of the capsule contents. The release time varies depending on wall material, thickness and void volume, and is usually less than one minute, typically less than 30 seconds and possibly much shorter than that, e.g. only a few seconds. The film material dissolves completely relatively soon after this, e.g. after a minute or so. The time for total dissolution varies depending on capsule size, content and wall thickness. This behaviour is to be contrasted to that of non-foamed film of the same thickness which dissolves more slowly in the mouth. The foamed film used in the present invention also has organoleptic properties in the mouth generally regarded as pleasant, providing a "melt-in-the-mouth" sensation, similar to that of eating rice paper.

Capsules in accordance with the invention find application as capsules intended for use in delivery of contents to the oral cavity of a user, for instance with the contents being in the form of a foodstuff such as confectionery or a medication such as a unit does of a throat-treatment liquid. In use of such capsules, the capsule wall starts to dissolve very rapidly after introduction to the mouth, possibly after sucking or chewing, releasing the contents into the mouth. The capsule wall material is pleasant to chew and dissolves completely in the mouth after a short time.

In a preferred aspect the invention thus provides an edible delivery capsule having an enclosing wall comprising a thermoplastic film of foamed modified cellulose material, preferably hydroxypropyl methyl cellulose, and capsule contents for release into the mouth of a consumer.

In contrast to known ingestible delivery capsules that are intended to be swallowed whole, with the capsule contents being released in the digestive tract of the consumer, the capsules of the invention are intended to be ruptured in the mouth of the consumer for release of contents into the mouth.

The enclosing wall is preferably made entirely or substantially entirely from thermoplastic film of foamed modified cellulose material, so that the entire wall can dissolve relatively rapidly in the mouth of a consumer.

The film may include optional colourings, e.g. in the form of known food dyes such as F D and C yellow number 5, optional flavourings, e.g. sweetenings, textures etc, in known manner. The film may also optionally include an acidulant material, such as citric acid, for improved mouth feel.

The capsule contents may be solid, e.g. in the form of a powder, granules, particles or a waxy solid etc, or liquid. In the case of liquid contents, because the film material is cold water soluble, these should contain little or no free or unbound water as otherwise the capsule wall will dissolve prematurely. However, bound water, e.g. as present in a carbohydrate solution such as an syrup, is acceptable, up to levels of about 40% of the weight of the liquid contents.

The capsules may optionally include one or more edible outer coatings, on top of the enclosing wall, e.g. in the form of a candy coating.

Although the film material is cold water soluble, the capsules are nevertheless found to be reasonably robust and will withstand a certain amount of handling, including being held in the hand, without the wall dissolving or rupturing prematurely.

The capsules may have a range of different sizes and shapes as appropriate dependent on intended usage. Capsules are typically generally spherical, ovoid, cylindrical etc. in shape. Typically the maximum dimension of the capsule is in the range 3mm to 50mm, but other sizes are possible. Where the capsule is intended to carry a unit dose, e.g. of a medication, the capsule can be appropriately sized to carry the desired dose.

The capsule may be compartmented, as described in WO 01/03676.

In a further aspect the invention thus provides a delivery capsule having at least two separate chambers, wherein at least part of the capsule wall comprises a thermoplastic film of a foamed modified cellulose material, preferably hydroxypropyl methyl cellulose.

In such compartmented capsules, some or all of the enclosing wall may comprise a thermoplastic film of foamed modified cellulose material, preferably hydroxypropyl methyl cellulose.

Capsules in accordance with the invention may be made in generally conventional manner, e.g. as disclosed in WO 97135537, WO 00/27367 and WO 01/03676.

The invention will be further described, by way of illustration, in the following example.

### Example

A foamed hydroxypropyl methyl cellulose film was made, having the following composition by weight:

| | |
|---|---|
| Hydroxypropyl methyl cellulose | 77% |
| Polyethylene glycol (plasticiser) | 23% |

The film was made in generally conventional manner. HPMC, in the form of a powder, was mixed with PEG and water to produce an aqueous solution, with stirring. The solution was gasified to a desired extent by addition of nitrogen gas in known manner.

The gasified solution was then fed to a feed hopper, including an elongate exit slot located a small distance above the upper surface of a moving conveyor belt adjacent one end thereof, with the slot extending perpendicularly with respect to the direction of movement of the belt. The feed arrangement geometry and speed of movement of the belt were such that a layer of liquid of desired thickness was applied to the belt and was moved on the belt away from the feed hopper, forming a film. The film was passed on the belt through a heating zone in which hot air heated the film, driving off water and so drying the film. The resulting dried, cast foamed HPMC film was removed from the belt and wound onto reels. The dried film had a thickness of about 150 microns, with a void volume of about 50%.

Edible delivery capsules were made from the film, e.g. as described in WO 97/35537, WO 00/27367 and WO 01/03676.

In one embodiment the capsules are in the form of a confectionery product, with the capsules being generally spherical with a diameter of about 20mm and containing a liquid chocolate or syrup formulation. On insertion into the mouth of a consumer, the foamed HPMC film starts to dissolve very rapidly, after a few seconds, releasing the liquid contents into the mouth. The film dissolves completely after about one minute, possibly assisted by sucking or chewing. The film has pleasant mouth-feel properties.

In another embodiment, the capsules are of similar form and size but contain a unitary dose of a medicated throat treatment liquid. In use, the liquid is rapidly released into the mouth of the consumer for contact with the throat region.

## Claims

1. A delivery capsule having an enclosing wall comprising a thermoplastic film of foamed modified cellulose material.

2. A capsule according to claim 1, wherein the film comprises hydroxypropyl methyl cellulose.

3. A capsule according to claim 1 or 2, wherein the foamed material has a void volume in the range 20 to 60% by volume.

4. A capsule according to claim 1, 2 or 3, wherein the film has a thickness in the range 50 to 200 microns.

5. An edible delivery capsule having an enclosing wall comprising a thermoplastic film of foamed modified cellulose material, and capsule contents for release into the mouth of a consumer.

6. A capsule according to claim 5, wherein the film comprises hydroxypropyl methyl cellulose.

7. A capsule according to any one of the preceding claims, further comprising one or more edible outer coatings, on top of the enclosing wall.

8. A delivery capsule having at least two separate chambers, wherein at least part of the capsule wall comprises a thermoplastic film of a foamed modified cellulose material.

9. A capsule according to claim 8, wherein the film comprises hydroxypropyl methyl cellulose.

## Patentansprüche

1. Verabreichungskapsel mit einer umschließenden Wand, umfassend einen thermoplastischen Film von geschäumtem modifizierten Cellulosematerial.

2. Kapsel gemäß Anspruch 1, wobei der Film Hydroxypropylmethylcellulose umfaßt.

3. Kapsel nach Anspruch 1 oder 2, wobei das geschäumte Material ein Hohlraumvolumen in dem Bereich von 20 bis 60 Vol.-% aufweist.

4. Kapsel nach Anspruch 1, 2 oder 3, wobei der Film eine Dicke in dem Bereich von 50 bis 200 µm aufweist.

5. Eßbare Verabreichungskapsel mit einer umschließenden Wand, umfassend einen thermoplastischen Film von geschäumtem modifizierten Cellulosematerial und Kapselinhalte zum Freisetzen in den Mund eines Verbrauchers.

6. Kapsel nach Anspruch 5, wobei der Film Hydroxypropylmethylcellulose umfaßt.

7. Kapsel nach einem der vorhergehenden Ansprüche, weiter umfassend eine oder mehrere eßbare Außenbeschichtungen oben auf der umschließenden Wand.

8. Verabreichungskapsel mit mindestens zwei getrennten Kammern, wobei mindestens ein Teil der Kapselwand einen thermoplastischen Film eines geschäumten modifizierten Cellulosematerials umfaßt.

9. Kapsel nach Anspruch 8, wobei der Film Hydroxypropylmethylcellulose umfaßt.

## Revendications

1. Gélule d'administration, comportant une paroi d'enceinte comprenant un film en cellulose modifiée expansé.

2. Gélule selon la revendication 1, dans laquelle le film comprend de l'hydroxypropyl méthyl cellulose.

3. Gélule selon l'une des revendications 1 ou 2, dans laquelle le matériau expansé présente un volume de vide dans la plage de 20 à 60 % en volume.

4. Gélule selon l'une des revendications 1, 2 ou 3, dans laquelle le film a une épaisseur dans la plage de 50 à 200 microns.

5. Gélule comestible d'administration ayant une paroi interne comprenant un film thermoplastique de cellulose modifiée expansé, et un contenu destiné à être libéré dans la bouche d'un consommateur.

6. Gélule selon la revendication 5, dans laquelle le film comprend de l'hydroxypropyl méthyl cellulose.

7. Gélule selon l'une quelconque des revendications précédentes, comprenant de plus un ou plusieurs revêtements comestibles, sur le dessus de la paroi d'enceinte.

8. Gélule d'administration ayant au moins deux chambres séparées, dans laquelle au moins une partie de la paroi de la gélule comprend un film thermoplastique en cellulose modifiée expansé.

9. Gélule selon la revendication 8, dans laquelle le film comprend de l'hydroxypropyl méthyl cellulose.
